# EUROPEAN PATENT SPECIFICATION

(11) **EP 1 412 747 B1**
(45) Date of publication and mention of the grant of the patent: **17.09.2008**
(21) Application number: 02748901.2
(22) Date of filing: 10.07.2002
(51) Int. Cl.: G01N 33/53, G01N 33/98

(54) **METHOD AND ANTIBODY FOR DETECTING ALCOHOL CONSUMPTION**
VERFAHREN UND ANTIKÖRPER ZUM NACHWEIS VON ALKOHOLKONSUM
METHODE ET ANTICORPS DESTINES A DECELER LA CONSOMMATION D'ALCOOL

(30) Priority: 11.07.2001 FI 20011517
(43) Date of publication of application: 28.04.2004
(73) Proprietor: Savolainen, Markku, 90220 Oulu (FI); Hannuksela, Minna, 90560 Oulu (FI); Liisanantti, Marja, 90800 Oulu (FI); Nissinen, Antti, 90250 Oulu (FI)
(72) Inventor: Savolainen, Markku, 90220 Oulu (FI); Hannuksela, Minna, 90560 Oulu (FI); Liisanantti, Marja, 90800 Oulu (FI); Nissinen, Antti, 90250 Oulu (FI)
(74) Representative: Boije-Backman, Solveig Magdalena
(86) International application number: PCT/FI2002/000626
(87) International publication number: WO 2003/010539

(56) References cited:
- DATABASE MEDLINE [Online] NATIONAL LIBRARY OF MEDICINE (NLM) SILLANAUKEE P. ET AL.: 'Acetaldehyde-modified hemoglobin as a marker of alcohol consumption: comparison of two new methods', XP002957978 Database accession no. 1613327 & THE JOURNAL OF LABORATORY AND CLINICAL MEDICINE vol. 120, no. 1, July 1992, pages 42 - 47
- DATABASE MEDLINE [Online] NATIONAL LIBRARY OF MEDICINE (NLM) VARGA A. ET AL.: 'Phosphatidylethanol in blood as a marker of ethanol consumption in healthy volunteers: comparison with other markers', XP002957979 Database accession no. 9835304 & ALCOHOLISM, CLINICAL AND EXPERIMENTAL RESEARCH vol. 22, no. 8, November 1998, pages 1832 - 1837
- DATABASE MEDLINE [Online] NATIONAL LIBRARY OF MEDICINE (NLM) GUNNARSSON T. ET AL.: 'Determination of phosphatidylethanol in blood from alcoholic males using high-performance liquid chromatography and evaporative light scattering or electrospray mass spectrometric detection', XP002957980 Database accession no. 9521560 & JOURNAL OF CHROMATOGRAPHY B, BIOMEDICAL SCIENCE AND APPLICATIONS vol. 705, no. 2, 13 February 1998, pages 243 - 249

## Description

### FIELD OF THE INVENTION

The invention relates to a method for detecting alcohol consumption from a sample. More specifically, the invention relates to a method for detecting alcohol consumption from a person's body sample, to an antibody used in the method, to a test kit including the antibody, and to a hybridoma producing said antibody. The invention further relates to a method for producing said antibody, to an antibody produced by the method, and to the use of the antibody.

### BACKGROUND OF THE INVENTION

Immoderate use of alcohol is a health hazard and a common problem in public health. A significant number of patients in health centres and hospitals are heavy users of alcohol, whose diseases may be alcohol-induced; the patients themselves, however, do not come out with their drinking problem. It may, however, be crucial that a drinking problem is revealed while planning a patient's treatment in order to be able to prevent alcohol-related problems from becoming more serious or at least to retard the process and, if necessary, to refer the patient to a more efficient treatment. Since patients do not usually reveal that they are heavy users of alcohol, a doctor must be capable of recognizing immoderate consumption in other ways, e.g. through laboratory tests.

A vast majority of alcohol (ethanol) consumed becomes oxidised to acetaldehyde and acetate and further to acetylcoenzyme A (AcCoA) in the body. Long-term heavy consumption of alcohol may cause liver damage (fatty liver, hepatitis alcoholica or cirrhosis of the liver), which may possibly be found as changes in liver enzyme values.

However, even immoderate use of alcohol cannot always be detected by ordinary laboratory tests, such as by determining liver enzyme values. Therefore, the aim has long been to provide better tests for this purpose. An alternative method for determining alcohol use has been described in Stibler 1991. In the publication, the amount of desialylated transferrin in a sample is detected cromatographically or immunologically. The use of alcohol increases the amount of this transferrin type and, even if the method is not very specific, it is probably the best indicator of alcohol use at the moment.

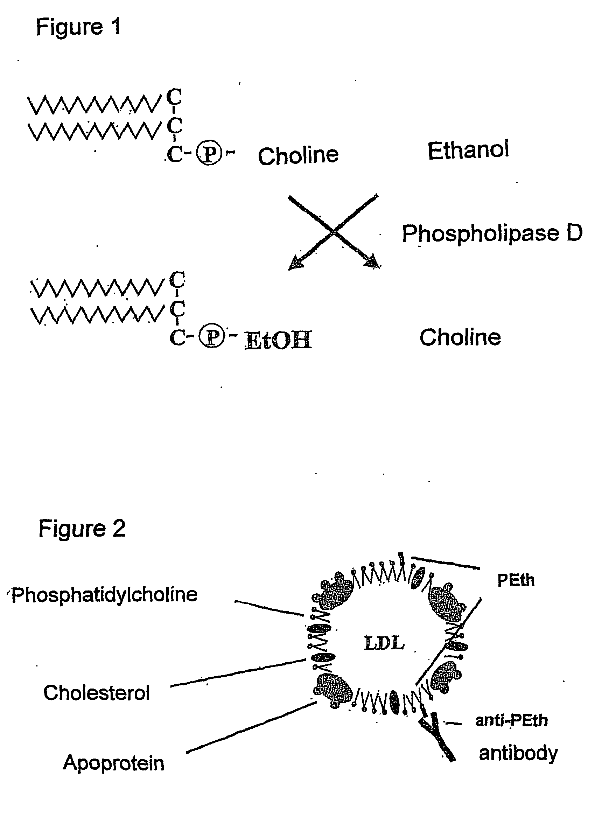

Another potential indicator of alcohol consumption is phosphatidylethanol (PEth). Phosphatidylethanol is an abnormal phospholipid, formed in the body only in the presence of alcohol. It has also been found in the blood from persons who have drunk alcohol (Varga et al. 1998). The reaction is catalyzed by phospholipase D, which replaces the choline part of phosphatidylcholine with ethanol. The reaction is shown in Figure 1. Phosphatidylethanol has been indirectly used as a marker of alcohol consumption in the method disclosed in publication WO 90/07008. In the method, blood lymphocytes are isolated and subsequently cultured with ethanol and a cell-stimulating substance, phorbol ester, the lymphocytes thus producing phosphatidylethanol. The resulting radioactive phosphatidylethanol is measured. According to the publication, a person prone to alcohol dependency has a larger than average capacity to produce phosphatidylethanol. The method is extremely laborious and the results obtained are contradictory. Gunnarsson et al. (1998) have described a simpler method wherein phosphatidylethanol is determined directly in blood, not in a lymphocyte culture. In the method, a lipid fraction is isolated from heparinized blood by hexane-isopropanol extraction and the presence of phosphatidylethanol is determined either mass spectrometrically or liquid chromatographically. Also these techniques are too complex in order to suit routine work.

Neither of the above-mentioned methods for assaying phosphatidylethanol is very reliable in detecting alcohol consumption. Furthermore, the large amount of work and complexity render the methods unsuitable for clinical routine work. The attempts to provide a reliable method suitable for routine laboratory work have been unsuccessful.

Immunoassay methods are often well suited for clinical laboratory tests. As far as phosphatidylalcohol is concerned, however, the problem has been the fact that it is difficult to produce an antibody against small molecules. Conventionally, small peptides or steroid hormones are attached to larger carrier proteins. Conventional carriers are, however, poorly suited for lipid antigens. Unexpectedly, however, an antibody has now been successfully produced against phosphatidylalcohol, enabling a considerably simpler assay for detecting heavy use of alcohol. It has thus been invented to detect heavy use of alcohol by immunologically determining phosphatidylalcohol formed in the body as a result of alcohol use.

Advantages of the method of the invention over prior art include reliability, small amount of work and quickness as well as the fact that no expensive chromatographic and/or spectrometric equipment are needed. The method is suitable to be used routinely also in small health care service (welfare for intoxicant abusers) units, enabling a patient's condition to be monitored in a flexible manner. The antibody of the invention may also be used in other research than that conducted on alcohol diseases.

### SUMMARY OF THE INVENTION

The present invention provides a method for detecting alcohol consumption from a person's body sample. The method of the invention is characterized in that phosphatidylalcohol comprising no more than four carbon atoms in its alcohol moiety and incorporated into a lipoprotein is determined from the sample by means of an antibody selectively recognizing said phosphatidylalcohol, the presence of phosphatidylalcohol in the sample indicating the person's alcohol consumption. The present invention also provides an antibody useful in the method, characterized in that it selectively recognizes said phosphatidylalcohol. The invention also provides a test kit, characterized in that it comprises an antibody selectively recognizing said phosphatidylalcohol. The invention further provides a hybridoma, characterized in that it produces an antibody selectively recognizing said phosphatidylalcohol.

One aspect of the invention further relates to a method for producing an antibody selectively recognising said phosphatidylalcohol, the method being characterized by immunizing an animal with the phosphatidylalcohol incorporated into a lipoprotein; and a) recovering the phosphatidylalcohol-recognizing antibody produced by the animal; or b) fusing cells of the immunized animal that produce the antibody with an immortal cell line in order to produce a hybridoma; selecting a hybridoma, producing the desired antibody; and recovering the antibody produced by the hybridoma. The invention further relates to an antibody produced by the above-mentioned method and to the use of the antibody of the invention in immunoassay.

### BRIEF DESCRIPTION OF THE DRAWINGS

Figure 1 describes formation of phosphatidylethanol (PEth) from phosphatidylcholine in a reaction catalyzed by phospholipase D,

Figure 2 describes binding of an anti-PEth antibody to a lipoprotein particle into which PEth has been incorporated,

Figure 3 describes determining PEth by an ELISA method, using the anti-PEth antibody and a second antibody recognizing an epitope of a lipoprotein, which is other than PEth,

Figure 4 describes the phospholipid specificity of the anti-PEth antibody,

Figure 5 shows the PEth concentrations of HDL particles in the plasma of heavy users of alcohol (black squares) and in the plasma of controls (white squares) as indicated using the anti-PEth antibody in radioimmunological assay, and

Figure 6 shows the PEth concentrations of HDL particles in the plasma of heavy users of alcohol (black squares) and in the plasma of controls (white squares) as indicated using the anti-PEth antibody in the ELISA assay method.

### DETAILED DESCRIPTION OF THE INVENTION

The idea underlying the present invention is that an antibody against phosphatidylalcohol has now been successfully produced. This was possible by incorporating phosphatidylalcohol into a lipoprotein and by using this phosphatidylalcohol incorporated into a lipoprotein as an antigen in immunization. This provides an antibody to the polar part of a phospholipid molecule, in this case phosphatidylalcohol. "Phosphatidylalcohol" is an ester of phosphatide acid, whose fatty acids in this case are usually the same as the fatty acids of naturally occurring phosphatidylcholine. "Lipoproteins" refer to a compound containing both protein and lipids. These occur both in cell membranes and as lipoprotein particles circulating in the body. The lipoprotein particles are well suited for serving as carriers of phosphatidylalcohol. In addition to phospholipids and proteins, the lipoprotein particles further comprise other components, such as triglycerides and cholesterol. The lipoprotein particles are classified e.g. on the basis of their density. For example, the following lipoprotein particles can be used in the preparation of an immunogen: VLDL (very low density lipoprotein), LDL (low density lipoprotein), HDL (high density lipoprotein), VHDL (very high density lipoprotein), IDL (intermediate density lipoprotein) or Lp(a) lipoprotein. The use of LDL, HDL and VLDL has yielded good results.

Phosphatidylalcohol, which is preferably phosphatidylethanol, is dissolved in a small amount of solvent and conveyed into a lipoprotein in an aqueous solution, the phosphatidylalcohol finding its way to among the lipids on the surface of the lipoprotein as the solvent concentration is diluted. The body of the animal to be immunized recognizes the polar residue of the small-molecule lipid that has appeared on the surface of the lipoprotein (in the case of ethanol an ethyl group) as a foreign substance, which enables an antibody to be obtained. Figure 2 shows_the binding of the antibody to phosphati-_{.}_ dylethanol on the surface of LDL lipoprotein. It is possible to Increase the anti-genity of an immunogen by using oxidised particles (undergone oxidation/subjected to oxidation). It is possible, for instance, to incorporate phosphatidylethanol more tightly into a lipoprotein by oxidising one fatty acid of phospholipid and, using the resulting aldehyde, to form a covalent bond between the protein part of the lipoprotein and the phosphatidylalcohol. It is also possible to increase immunogenity (and even the semblance of an alcoholic's lipoprotein) by modifying the lipoprotein particle also otherwise, for instance by ethylating the lipoprotein e.g. by acetaldehyde treatment prior to adding PEth (or after it). It is also possible to desialylate the lipoproteins, i.e. remove sialic acid therefrom, in which case they resemble an alcoholic's lipoproteins more closely.

The above-described phosphatidylalcohol incorporated into a lipoprotein can be used as an antigen in a manner known *per se* in the preparation of the antibody of the invention recognizing phosphatidylalcohol. An "antibody recognizing phosphatidylalcohol" refers to an antibody capable of selectively binding to phosphatidylalcohol, including fragments of said antibody also capable of selectively binding to phosphatidylalcohol. Such fragments include e.g. F(ab')₂ and F(ab).

An immunologically reactive animal is immunized with phosphatidylalcohol incorporated into a lipoprotein and the immune response formed against an antigen is tested. A polyclonal antibody can be recovered from animals giving a positive response to the antigen, and purified using methods known to those skilled in the art. In another embodiment of the invention, a monoclonal antibody is prepared. After immunizations, the antibody-producing cells of the animals giving a positive response to the antigen, usually spleen cells, are fused with an immortal cell line in order to obtain a hybridoma producing the antibody. The antibody is isolated from the culture medium of the positive hybridomas, and purified using methods commonly known to those skilled in the art.

If desired, a fragment recognizing phosphatidylalcohol can be isolated from the antibody prepared. Alternatively, such a fragment can be produced using methods of gene technology. The antibody, including the fragments, can be further modified e.g. by incorporating a label, such as a radioactive isotope, a metal particle or a fluorescent or enzyme label (e.g. ³H, colloidal gold, rhodamine, fluorescein, peroxidase, alcalic phosphatase) thereto in manners known per se.

The antibody of the invention now enables phosphatidylalcohol, and in particular phosphatidylalcohol incorporated into a lipoprotein, to be detected immunologically. Phosphatidylalcohol to be detected immunologically is preferably phosphatidylethanol and, in particular, phosphatidylethanol incorporated into a lipoprotein. The immunological method for determining phosphatidylalcohol is intended particularly for detecting and monitoring heavy use of alcohol in a reliable manner. Consumption exceeding 24 doses of alcohol in men and 16 doses of alcohol in women per week or, alternatively, more than 7 doses in men and more than 5 doses in women at a single occasion can be considered as heavy use of alcohol. One dose corresponds with about 12 grams of pure ethanol. The method can also be utilized in finding and studying alcohol diseases. In the presence of alcohol in the body, an alcohol molecule incorporates into phospholipids in a reaction catalyzed by phospholipase D wherein ordinarily water incorporates. The amount of phosphatidylalcohol in the body correlates with alcohol consumption, and phosphatidylalcohol remains in the body for at least 2 to 4 days after consumption, maybe longer.

The antibody recognizing phosphatidylalcohol can be used in any immunological assay based on detecting a complex between an antigen and an antibody. This complex can be detected either directly or indirectly by using secondary antibodies. A solid carrier is often used in immunoassays, in which case either the antibody or the antigen is bound to the carrier. The antibody bound to the carrier may also be a so-called capture antibody used as an intermediate conjugate in order to bind a primary antibody to the carrier.

In one embodiment, a radioimmunological assay (RIA) is used which can be carried out such that the antibody against phosphatidylalcohol is, directly or by means of an intermediate conjugate, attached to the carrier. The lipoproteins of a patient's sample are marked by a radio isotope in a manner known per se and the samples are left to react with the antibody attached to the carrier. The binding of the lipoproteins containing phosphatidylalcohol to the carrier is detected by measuring radioactivity.

A highly suitable immunoassay for detecting alcohol consumption is an assay called ELISA (Enzyme Linked immune Sorbent Assay), wherein an antibody labelled with an enzyme is used. An enzyme marker can be incorporated either directly into the antibody of the invention or, alternatively, to a secondary antibody. One embodiment suitable for ELISA can be implemented by attaching the antibody of the invention directly or by means of an intermediate conjugate to a solid carrier by leaving it to react with a sample and by detecting the bound phosphatidylalcohol incorporated into a lipoprotein by means of a second antibody recognizing an epitope of said lipoprotein, the epitope being other than phosphatidylalcohol. An enzyme marker can be attached to said second antibody or said second antibody can be detected by means of a secondary antibody containing the marker. A marker is usually detected by adding a substrate to the enzyme and by detecting the colour of the product produced.

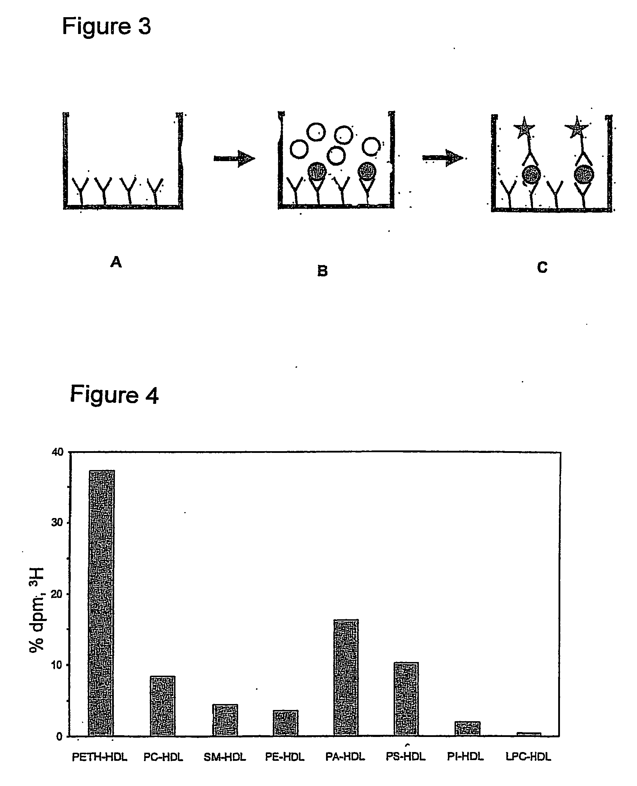

Figure 3 shows determining the phosphatidylethanol content from a sample by using the ELISA method wherein, in addition to the antibody provided against phosphatidylethanol, a second antibody is used which recognizes the protein part of a lipoprotein containing phosphatidylethanol. A) describes the attachment of an anti-PEth antibody to the bottom of the wells of a well plate. B) describes the addition of the sample onto the well plate. An ordinary HDL is an open circle while an HDL containing PEth is a grey circle. C) shows the quantification of the amount of HDL particles containing PEth with a labelled antibody directed against apoprotein A-I.

The second antibody used in the assay is usually directed against the protein part of a lipoprotein containing phosphatidylalcohol. These so-called apolipoproteins include e.g. apoAl, apoAll, apoB100, apoB-48, apoCI, apoCII, apoCIII, apoD, apoE and apoJ. An LDL particle contains only one protein apoB-100, which is also contained in VLDL particles. HDL particles do not contain apoB-100 but mostly apoAl and apoAll.

In addition to the antibody of the invention, the test kit of the invention may also include other reagents and devices necessary for the immunoassay of phosphatidylalcohol, such as other, possibly labelled antibodies and reagents to be used in the assay in order to detect a marker, as well as necessary buffers and/or carriers, such as microtiter plates to which reagents, such as an antibody, have possibly been attached already.

A body sample to be examined using an immunological assay method may be e.g. blood, plasma, serum, saliva, tissue or amnical fluid or some other part or fluid containing phosphatidylalcohol. The sample may also be a medico-legal sample, a *post mortem* sample or a sample taken from tissues of an embryo or a foetus. Most suitable for ELISA and RIA assays are fluid samples, such as plasma, serum and blood, which can be used in the assay as such, without lipid fraction extraction or dissolution.

Instead of phosphatidylethanol, a phosphatidyl of another alcohol may be incorporated into a lipoprotein in order to obtain an antibody against the phosphatidyl of this alcohol. Alcohols that may be used include straight-chain alcohols comprising no more than eight, preferably 1 to 4, carbon atoms, i.e. alcohols that are converted into phosphatidylalcohols in a reaction catalyzed by phospholipase D. Different antibodies recognizing phosphatidylalcohols may thus also be used in determining the quality of the alcohol consumed by a patient in order to indicate use of impotable alcohols, such as ethylene glycol, isopropyl alcohol, methanol and possibly also propanol, butanol and pentanol, enabling appropriate treatment.

Other embodiments of the invention further include immunological methods, such as antibody columns, Western blot and immuno-histological methods. Naturally, antibodies produced against phosphatidylalcohols may also be used in biochemical basic research, such as research on lipid metabolism. Indirectly, they may also be used for detecting the activity and location of phospholipase D e.g. from cell or tissue cultures or histological samples.

The invention will be explained by means of the following non-restrictive examples.

### Example 1

### Preparation of immunogen

Lipoproteins were isolated from the plasma of healthy humans. The blood samples were obtained after an overnight fast and the plasma was separated by centrifugation. The lipoproteins were isolated from the plasma by sequential ultracentifugation on the basis of their density. A VLDL fraction was isolated from a density of 1.006 g/ml, an LDL fraction from a density of 1.019 to 1.063 g/ml and an HDL from a density of 1.063 to 1.21 g/ml. After centrifugation, the lipoprotein fractions were dialyzed against 0.15 M NaCl, 0.01 % EDTA, pH 7.4 or PBS, pH 7.4 at +4°C.

The phospholipid concentration of the lipoprotein particles was measured and the necessary amount of phosphatidylethanol was calculated such that its final content was 5% of the total amount of phospholipids. Phosphatidylethanol, which was 1,2-dioleoyl-sn-glycero-3-phosphoethanol sodium salt (Avanti Polar Lipids, USA), was incorporated into human isolated HDL or LDL particles (5% of lipoprotein phospholipids) in accordance with the following method: Said phosphatidylethanol, which was dissolved in chloroform (10 mg/ml) was first dried under nitrogen and further under vacuum for overnight at room temperature. The dry lipid film was then dissolved in a small volume of ethanol and added (50 to 100 µl) drop-wise into about 1 ml of a water salt solution (0.15 M NaCl, 0.01 % EDTA, pH 7.4) containing the HDL or LDL particles. The solutions were incubated overnight in cold (+4°C) and then adjusted to a density of 1.21 g/ml (HDL) or 1.063 g/ml (LDL) with NaBr solution and centrifuged at 114 000 g for 18 h at 15°C. The centrifuged lipoprotein fractions were dialyzed against 0.5 M NaCl, 0.01 % EDTA, pH 7.4. The phosphatidylethanol-containing lipoprotein particles obtained were used as immunogens.

**Example 2**

**Preparation of antibody**

A monoclonal anti-PEth antibody was prepared using standard protocols (Oi & Herzenberger 1980). Six-week-old female Balb/c mice were immunized at intervals of three weeks with a phosphatidylethanol antigen incorporated into LDL particles, which was prepared according to Example 1. After second immunization, the positive serum samples were identified using a direct ELISA assay wherein the antigen, i.e. PEth lipoprotein, was bound to wells, the serum to be tested was added, washing was carried out and bound antibody was determined using an anti-mouse immunoglobulin antibody, which was labelled with horseradish peroxidase. The spleens of the mice that had given a positive response to the antigen were isolated and spleen cells were fused with mouse myeloma cell line P3-X63-Ag8.653 (ATCC CRL-1580, American Type Culture Collection, USA) using polyethylene glycol (PEG 4000, Gibco, UK). Hybridoma cells were selected on a well plate in HAT medium [DMEM, high glucose (Gibco, UK), 10% NCTC-135 (Gibco), 20% FBS (Bio-clear, UK), 5% HFCS (Roche Molecular Biochemicals GmbH, Germany), HAT supplement (Gibco) and penicillin/streptomycin solution (Gibco)]. The hybridoma cells were screened using the direct ELISA against the antigen, as described above. The positive wells were cloned using a limiting dilution method and the wells having a single colony only were again screened using the direct ELISA. The culture media of the positive hybridomas were collected and the monoclonal antibody was purified with protein-G-Sepharose-affinity chromatography (Pharmacia, Sweden). In the following tests, the class of the hybridoma antibody used was determined as IgG_{2b} by using antibody capture on anti-Ig antibody wells (Biotop, Finland).

**Example 3**

**Determining phospholipid specifity of anti-PEth antibody**

A radioimmunoassay was carried out. Wells coated with rabbit anti-mouse IgG antibody (Wallac, Delfia wells, Finland) were washed twice with PBS solution (pH 7.4), blocked with 1 % BSA-PBS solution, 300 µl/well for 30 minutes at +20°C in a humid chamber, mixing, and washed again twice with PBS solution. 50 µl/well of the monoclonal mouse anti-PEth antibody prepared in Example 2 and diluted 1:50 in 1 % BSA-PBS solution, the amount of antibody then being 1.4 µg/ml, was added to the wells and incubated for six hours at +4°C in a humid chamber, mixing; washed twice with PBS solution. 50 µl of 5-% phospholipid HDL solutions which were diluted in PBS solution and whose HDL was labelled with ³H cholesterol (0.5 mg/ml of protein, 0.08.mg/ml of added phospholipid) were added as antigen solutions to the wells. The follow ing phospholipid solutions were tested:

PEth-HDL: 5% phosphatidylethanol added to ³H-HDL solution

PC-HDL: 5% phosphatidylcholine added to ³H-HDL solution

SM-HDL: 5% sphingomyelin added to ³H-HDL solution

PE-HDL: 5% phosphatidylethanol amine added to ³H-HDL solution

PA-HDL: 5% phosphatide acid added to ³H-HDL solution

PS-HDL: 5% phosphatidyl serine added to ³H-HDL solution

PI-HDL: 5 % phosphatidylinositol added to ³H-HDL solution

LPC-HDL: 5% lysophosphatidylcholine added to ³H-HDL solution

Incubation in a cold room overnight, mixing.

The wells were washed four times and dabbed dry. Next, the wells were cut apart using a guillotine cutter and dropped into 2 ml of scintillation liquid (OptiPhase Highsafe 3, Wallac). After vortexing, radioactivity was measured by a beta counter (Wallac) using ³H programme and a 5-minute measurement time. The results are shown as the amount (% dpm) of bound radioactivity in Figure 4. The results show that the anti-PEth antibody used recognizes specifically the phosphatidylethanol incorporated into a lipoprotein.

**Example 4**

**Radioimmunoassay (RIA) for determining phosphatidylethanol**

A group of heavy users of alcohol whose alcohol consumption on average was 96 doses of alcohol (ranging between 55 and 137 doses) per week and who had referred for detoxification as well as a group of controls whose alcohol consumption on average was 9 doses of alcohol (ranging between 4 and 15 doses) per week were used as testees. The testees had no (other) noticeable health problems. Venous blood samples were taken from the testees after an overnight fast and plasmas were separated by centrifugation. Lipoprotein fractions were separated from the plasma as described in Example 1. The isolated HDL fractions were labelled with ³H cholesterol ester (Tollefson & Albers, 1986).

Well plates wish detachable wells as 8-well strips (NUNC Lock Well MaxiSorp) were used in the assay. The wells of microtiter plates were coated with goat anti-mouse-IgG (H+L) antibody (Zymed Laboratories, USA) 20 µg/ml (diluted in PBS solution), 50 µl/well and incubated for 24 h at +4°C. Washing was carried out twice with PBS solution, after which the mouse monoclonal anti-PEth antibody (100 µl/well of a 1:50 dilution in PBS solution of the stem solution 43 ng/µl) prepared in Example 2 was added and the antibody was allowed to bind for one hour at room temperature. The wells were washed twice with PBS solution and blocked for two hours at room temperature with 1% BSA-PBS solution at a volume of 300 µl/well, after which the blocking was removed.

HDL fractions labelled with ³H cholesterol ester isolated from the plasma of heavy users of alcohol and the controls as well as standard samples each 50 µl/well were incubated in a cold room overnight. The samples were diluted in a sample buffer (1xPBS, 1% BSA, 0.5% TritonX-100, 1 mM EDTA) to a concentration of 0.5 mg/ml of protein. The standard samples used were 0%, 1% and 10% PEth-HDL, which was made using a solution comprising pooled HDL fractions from different controls. Next, the wells were washed four times with PBS solutions containing 0.5% of TritonX-100, and again twice with PBS.

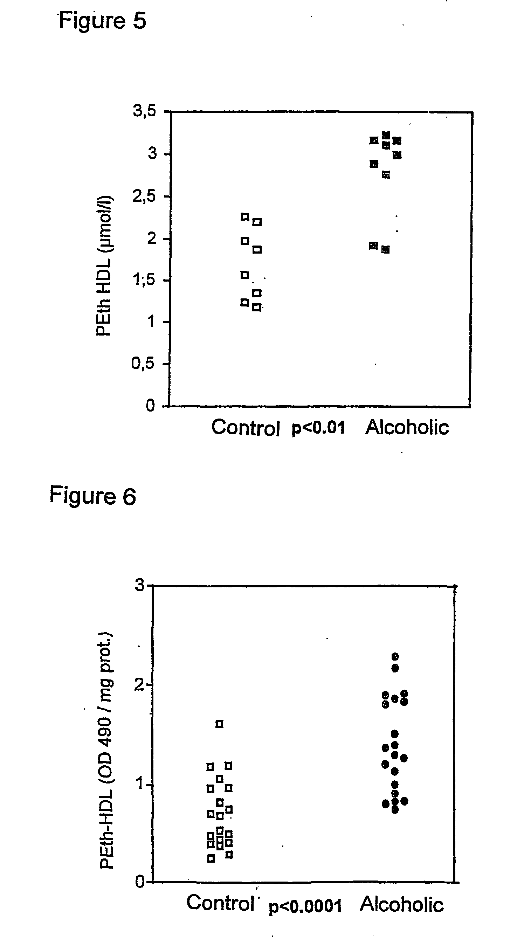

The wells were dabbed dry and dropped into scintillation bottles each comprising 2 ml of scintillation liquid (OptiPhase Highsafe 3, PerkinElmer, Wallac, Finland), vortexed for 30 seconds and measured for radioactivity using a Wallac Rack-beta scintillation counter (³H count). The PEth concentration contained in the samples was calculated using standards. The results are shown in Figure 5. The tests showed that the phosphatidylethanol content in the HDL particles of heavy users of alcohol (2.75 ± 0.53 µmol/l) was significantly higher than in the control group (1.70 ± 0.43 µmol/l) (p<0.01, Student's t-test).

**Example 5**

### ELISA method for determining phosphatidylethanol

A group of heavy users of alcohol whose alcohol consumption on average was 81 doses of alcohol (ranging between 44 and 117 doses) per week and who had referred for detoxification as well as a group of controls whose alcohol consumption on average was 13 doses of alcohol (ranging between 1 and 25 doses) per week were used as testees. The testees had no (other) noticeable health problems. Venous blood samples were taken from the testees after an overnight fast and plasmas were separated by centrifugation.

Wells of a microtiter plate (Delfia, Wallac, Finland) were coated with rabbit anti-mouse-IgG(H+L) antibody. The wells were then washed twice with PBS solution. The mouse monoclonal anti-PEth antibody (100 µl/well of a 1:50 dilution in PBS solution of the stem solution 43 ng/µl) prepared in Example 2 was added to each well and left to react for one hour at room temperature. The wells were washed twice with PBS solution and blocked with 1 % BSA-PBS solution about 350 µl/well for half an hour at room temperature, and washed again twice with PBS solution.

Fast plasma diluted in 1:10 PBS solution was then added 100 µl/well, two parallel ones from each sample. These were left to react at +4°C overnight. The wells were washed twice with PBS solution and added to sheep anti-human ApoAI antibody (Roche Diagnostic, Germany) diluted in 1:500 1% BSA-PBS solution 100 µl/well and left to react at room temperature for one hour. The wells were then washed three times with PBS solution. Per oxidase labelled donkey anti-sheep-IgG(Fab)-POD antibody (Roche Diagnostic, Germany) diluted 1:1000 in 1 % BSA-PBS solution was then added 100 µl/well and left to react at room temperature for one hour. After the PBS washings, a peroxidase reaction was conducted using 1,2-diaminobenzene (OPD) (Sigma) as a substrate. An OPD tablet was dissolved just before use in 20 ml of buffer and added 100 µl/well and incubated for 5 to 15 minutes at room temperature in dark. The reaction was stopped using 3 M sulphuric acid 50 µl/well. Absorbance was measured at a wavelength of 490 nm.

The results are shown in Figure 6. The results show that the phosphatidylethanol content in the HDL particles of heavy users of alcohol was significantly higher than in the control group (p<0.0001).

The VLDL and LDL particles bound by the mouse anti-human-PEth-IgG_{2b} antibody prepared in Example 2 and containing phosphatidylethanol were detected, correspondingly, from the plasma by using an anti-human-apoB100 antibody (Roche Diagnostic) as a second antibody. The results obtained were in line with those obtained in the tests conducted using anti-human-ApoAl antibody.

### REFERENCE LIST

Gunnarsson,T., Karlsson, A., Hansson, P., Johnson, G., Alling, C., & Odham, G. (1998) Determination of phosphatidylethanol in blood from alcoholic males using high-performance liquid chromatography and evaporative light scattering or electrospray mass spectrometric detection, J.Chromatogr.B Biomed.Sci.Appl. 705: pp. 243 to 249.
Oi, V. T. & Herzenberger, L. A. (1980) Immunoglobulin-producing hybrid cell lines. In Selected Methods in Cellular Immunology, B.B.Mishell and Shiigi,S.M., editors. Freeman, San Francisco, pp. 351 to 372.
Stibler H. (1991) Carbohydrate-deficient transferrin in serum: A new marker of potentially harmful alcohol consumption reviewed, Clin. Chem. 12: pp. 2029 to 2037.
Tollefson, J. H. & Albers, J. J. (1986) Isolation, characterization, and assay of plasma lipid transfer proteins, Methods Enzymol. 129: pp. 797 to 816.
Varga, A., Hansson, P., Lundqvist, C., & Alling, C. (1998) Phosphatidylethanol in blood as a marker of ethanol consumption in healthy volunteers: comparison with other markers, Alcohol Clin.Exp.Res. 22: pp. 1832 to 1837.

## Claims

1. A method for detecting alcohol consumption from a person's body sample, **characterized in that** phosphatidylalcohol comprising no more than four carbon atoms in its alcohol moiety and incorporated into a lipoprotein is determined from the sample by means of an antibody selectively recognizing said phosphatidylalcohol, the presence of phosphatidylalcohol in the sample indicating the person's alcohol consumption.

2. A method as claimed in claim 1, **characterized in that** in addition to said antibody, a second antibody recognizing an epitope of said lipoprotein is used, the epitope being other than phosphatidylalcohol.

3. A method as claimed in claim 2, **characterized in that** the second antibody recognizes an epitope of lipoprotein particle HDL, LDL or VLDL.

4. A method as claimed in claim 3, **characterized in that** the second antibody recognizes lipoprotein particle protein apoAI or apoB100.

5. A method as claimed in any one of claims 1 to 4, **characterized in that** the method is an ELISA assay method.

6. A method as claimed in any one of the preceding claims, **characterized in that** a monoclonal antibody is used.

7. A method as claimed in claim 6, **characterized in that** the phosphatidylalcohol is determined in a blood, plasma or serum sample.

8. A method as claimed in any one of the preceding claims, **characterized in that** phosphatidylethanol is determined.

9. An antibody, **characterized in that** it selectively recognizes phosphatidylalcohol comprising no more than four carbon atoms in its alcohol moiety and incorporated into a lipoprotein.

10. An antibody as claimed in claim 9, **characterized in that** it is a monoclonal antibody.

11. A test kit, **characterized in that** it comprises an antibody selectively recognizing phosphatidylalcohol comprising no more than four carbon atoms in its alcohol moiety and incorporated into a lipoprotein.

12. A hybridoma, **characterized in that** it produces an antibody selectively recognizing phosphatidylalcohol comprising no more than four carbon atoms in its alcohol moiety and incorporated into a lipoprotein.

13. A method for preparing an antibody selectively recognizing phosphatidylalcohol comprising no more than four carbon atoms in its alcohol moiety, **characterized by**
immunizing a non-human animal with phosphatidylalcohol comprising no more than four carbon atoms in its alcohol moiety incorporated into a lipoprotein; and
a) recovering the phosphatidylalcohol-recognizing antibody produced by the animal; or
b) fusing cells of the immunized animal that produce the antibody with an immortal cell line in order to produce a hybridoma; selecting a hybridoma producing the desired antibody; and recovering the antibody produced by the hybridoma.

14. A method as claimed in claim 13, **characterized by** immunizing with said phosphatidylalcohol incorporated into lipoprotein particle HDL, LDL or VLDL.

15. An antibody produced by the method of claim 13 or 14.

16. The use of the antibody of any one of claims 9, 10 or 15 in an immunoassay.

## Patentansprüche

1. Verfahren zum Nachweisen von Alkoholkonsum aus einer Körperprobe einer Person, **dadurch gekennzeichnet, dass** Phosphatidylalkohol, der nicht mehr als 4 Kohlenstoffatome in seiner Alkoholkomponente enthält und einem Lipoprotein inkorporiert ist, aus der Probe mittels eines Antikörpers bestimmt wird, der selektiv den Phosphatidylalkohol erkennt, wobei das Vorliegen von Phosphatidylalkohol in der Probe den Alkoholkonsum der Person anzeigt.

2. Verfahren wie in Anspruch 1 beansprucht, **dadurch gekennzeichnet, dass** zusätzlich zu dem Antikörper ein zweiter Antikörper, der ein Epitop des Lipoproteins erkennt, verwendet wird, wobei das Epitop von dem Phosphatidylalkohol verschieden ist.

3. Verfahren wie in Anspruch 2 beansprucht, **dadurch gekennzeichnet, dass** der zweite Antikörper ein Epitop eines HDL-, LDL- oder VLDL- Lipoproteinteilchens erkennt.

4. Verfahren wie in Anspruch 3 beansprucht, **dadurch gekennzeichnet, dass** der zweite Antikörper ein apoAI- oder apoB100-Lipoproteinteilchen erkennt.

5. Verfahren wie in einem der Ansprüche 1 bis 4 beansprucht, **dadurch gekennzeichnet, dass** das Verfahren ein ELlSA-Assay-Verfahren ist.

6. Verfahren wie in einem der vorhergehenden Ansprüche beansprucht, **dadurch gekennzeichnet, dass** ein monoklonaler Antikörper verwendet wird.

7. Verfahren wie in Anspruch 6 beansprucht, **dadurch gekennzeichnet, dass** der Phosphatidylalkohol in einer Blut-, Plasma- oder Serumprobe bestimmt wird.

8. Verfahren wie in einem der vorhergehenden Ansprüche beansprucht, **dadurch gekennzeichnet, dass** Phosphatidylethanol bestimmt wird.

9. Antikörper, **dadurch gekennzeichnet, dass** er selektiv Phosphatidylalkohol, der nicht mehr als vier Kohlenstoffatome in seiner Alkoholkomponente enthält und in einem Lipoprotein inkorporiert ist, erkennt.

10. Antikörper wie in Anspruch 9 beansprucht, **dadurch gekennzeichnet, dass** er ein monoklonaler Antikörper ist.

11. Testkit, **dadurch gekennzeichnet, dass** er einen Antikörper enthält, der selektiv Phosphatidylalkohol, der nicht mehr als vier Kohlenstoffatome in seiner Alkoholkomponente enthält und in einem Lipoprotein inkorporiert ist, erkennt.

12. Hybridom, **dadurch gekennzeichnet, dass** es einen Antikörper produziert, der selektiv Phosphatidylalkohol, der nicht mehr als vier Kohlenstoffatome in seiner Alkoholkomponente enthält und in einem Lipoprotein inkorporiert ist, erkennt.

13. Verfahren zum Herstellen eines Antikörpers, der selektiv Phosphatidylalkohol, der nicht mehr als vier Kohlenstoffatome in seiner Alkoholkomponente enthält, erkennt, **gekennzeichnet durch**
immunisieren eines nicht-menschlichen Tieres mit Phosphatidylalkohol, der nicht mehr als vier Kohlenstoffatome in seiner Alkoholkomponente enthält und in einem Lipoprotein inkorporiert ist; und
(a) Isolieren des von dem Tier produzierten Phosphatidylalkohol erkennenden Antikörpers; oder
(b) Verschmelzen von Zellen des immunisierten Tieres, das den Antikörper produziert, mit einer unsterblichen Zelllinie, um ein Hybridom zu produzieren; Auswählen eines den erwünschten Antikörper produzierenden Hybridoms; und Isolieren des von dem Hybridom produzierten Antikörpers.

14. Verfahren gemäß Anspruch 13, **gekennzeichnet durch** Immunisieren mit dem in einem HDL-, LDL- oder VLDL-Lipoproteinteilchen inkorporierten Phosphatidylalkohol.

15. Antikörper, hergestellt durch das Verfahren nach Anspruch 13 oder 14.

16. Verwendung des Antikörpers nach einem der Ansprüche 9, 10 oder 15 in einem Immunoassay.

## Revendications

1. Procédé pour détecter la consommation d'alcool à partir d'un échantillon du corps d'une personne, **caractérisé en ce que** le phosphatidylalcool ne comprenant pas plus de quatre atomes de carbone dans son groupe fonctionnel alcool et étant incorporé dans une lipoprotéine est déterminé à partir de l'échantillon au moyen d'un anticorps identifiant sélectivement ledit phosphatidylalcool, la présence de phosphatidylalcool dans l'échantillon indiquant la consommation d'alcool de la personne.

2. Procédé selon la revendication 1, **caractérisé en ce qu'**en plus dudit anticorps, un deuxième anticorps identifiant un déterminant antigénique de ladite lipoprotéine est utilisé, le déterminant antigénique étant autre que le phosphatidylalcool.

3. Procédé selon la revendication 2, **caractérisé en ce que** le deuxième anticorps identifie un déterminant antigénique de la particule de lipoprotéine HDL, LDL ou VLDL.

4. Procédé selon la revendication 3, **caractérisé en ce que** le deuxième anticorps identifie la particule de protéine apoAI ou apoB100 de lipoprotéine.

5. Procédé selon l'une des revendications 1 à 4, **caractérisé en ce que** le procédé est une technique de dosage ELISA (dosage d'immunosorption liée à enzyme).

6. Procédé selon l'une des revendications précédentes, **caractérisé en ce qu'**un anticorps monoclonal est utilisé.

7. Procédé selon la revendication 6, **caractérisé en ce que** le phosphatidylalcool est déterminé dans un échantillon de sang, de plasma ou de sérum.

8. Procédé selon l'une des revendications précédentes, **caractérisé en ce que** le phosphatidyléthanol est déterminé.

9. Anticorps **caractérisé en ce qu'**il identifie sélectivement le phosphatidylalcool ne comprenant pas plus de quatre atomes de carbone dans son groupe fonctionnel alcool et **en ce qu'**il est incorporé dans une lipoprotéine.

10. Anticorps selon la revendication 9, **caractérisé en ce qu'**il est un anticorps monoclonal.

11. Kit d'essai, **caractérisé en ce qu'**il comprend un anticorps identifiant sélectivement le phosphatidylalcool ne comprenant pas plus de quatre atomes de carbone dans son groupe fonctionnel alcool et étant incorporé dans une lipoprotéine.

12. Hybridome, **caractérisé en ce qu'**il produit un anticorps identifiant de manière sélective le phosphatidylalcool ne comprenant pas plus de quatre atomes de carbone dans son groupe fonctionnel alcool et étant incorporé dans une lipoprotéine.

13. Procédé pour préparer un anticorps identifiant sélectivement le phosphatidylalcool ne comprenant pas plus de quatre atomes de carbone dans son groupe fonctionnel alcool, **caractérisé :**
**par** le fait de réaliser une immunisation d'un animal non humain avec du phosphatidylalcool ne comprenant pas plus de quatre atomes de carbone dans son groupe fonctionnel alcool, incorporé dans une lipoprotéine ; et
a) par le fait de récupérer l'anticorps identifiant le phosphatidylalcool produit par l'animal ; ou
b) par le fait de fusionner les cellules de l'animal immunisé que produit l'anticorps avec lignée cellulaire immortelle afin de produire un hybridome ; par le fait de choisir un hybridome produisant l'anticorps désiré ; et par le fait de récupérer l'anticorps produit par l'hybridome.

14. Procédé selon la revendication 13, **caractérisé par** le fait de réaliser une immunisation avec ledit phosphatidylalcool incorporé dans la particule de lipoprotéine HDL, LDL ou VLDL.

15. Anticorps produit par le procédé selon les revendications 13 ou 14.

16. Utilisation de l'anticorps de l'une des revendications 9, 10 ou 15 dans un immunoessai.
